# FASCICULE DE BREVET EUROPEEN

(11) **EP 0 640 355 B1**
(45) Date de publication et mention de la délivrance du brevet: **26.01.2000**
(21) Numéro de dépôt: 94401749.0
(22) Date de dépôt: 29.07.1994
(51) Int. Cl.: A61M 16/04

(54) **Tube d'assistance respiratoire**
Tubus zur Atmungsunterstützung
Tube for respiratory assistance

(30) Priorité: 26.08.1993 FR 9310264
(43) Date de publication de la demande: 01.03.1995
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- EP-A- 0 074 809
- EP-A- 0 152 694
- EP-A- 0 245 142
- DE-A- 2 353 153
- GB-A- 1 171 439
- US-A- 3 707 151
- US-A- 5 235 973
- US-A- 5 318 021

## Description

La présente invention a pour objet un tube d'assistance respiratoire, utilisable sur des patients dont la respiration spontanée est absente ou insuffisante, et applicable en tant que, par exemple, sonde endotrachéale oro-nasale, sonde endotrachéale pédiatrique, sonde de monitorage des gaz, sonde endobronchique, sonde nasopharyngée, sonde d'intubation anatomique pour enfant, sonde de Cole néonatale, sonde canule de Guedel, sonde nasale d'oxygénothérapie, ou dispositif de traitement d'apnée du sommeil.

On connaît déjà des appareils d'assistance respiratoire comportant des moyens pour amener du gaz respiratoire dans les poumons d'un patient et, éventuellement, pour en évacuer ledit gaz, par l'intermédiaire d'un tube ou sonde d'assistance respiratoire dont l'extrémité distale est destinée à être introduite dans la trachée du patient. Dans ce cas, il est d'usage de disposer, à l'extrémité distale du tube, un ballonnet gonflable destiné à assurer l'étanchéité entre le tube et la trachée. Ce ballonnet est introduit à l'état dégonflé dans la trachée, puis gonflé une fois en place dans celle-ci. Pour le gonflement du ballonnet, on prévoit du côté proximal du tube, un générateur de gaz de gonflage, généralement incorporé au générateur de gaz respiratoire. La pression de gonflage du ballonnet, indépendante de la pression du gaz respiratoire insufflé dans les poumons du patient, est déterminée pour que le ballonnet s'applique de façon continue contre les parois de la trachée et qu'il en résulte une étanchéité suffisante entre le tube d'assistance respiratoire et la trachée.

Malgré toutes les précautions que l'on peut prendre (comme de limiter la pression de gonflage à la plus petite valeur compatible avec l'étanchéité indispensable), la pression d'appui exercée par le ballonnet contre les parois de la trachée est traumatisante pour celles-ci. Il peut en résulter des conséquences graves pour le patient, telles que la nécrose des tissus des parois de la trachée.

Pour remédier à cet inconvénient et permettre de supprimer, ou à tout le moins de fortement diminuer, le traumatisme trachéal dû aux ballonnets gonflables des tubes d'assistance respiratoire introduits dans une trachée, le document US-A-3 707 151 décrit un tube d'assistance respiratoire, dont l'extrémité distale est destinée à être introduite dans la trachée d'un patient et est munie d'un ballonnet gonflable pour assurer l'étanchéité entre ledit tube d'assistance respiratoire et ladite trachée, et dont l'extrémité proximale est destinée à être reliée à des moyens pour amener du gaz respiratoire dans les poumons du patient, par l'intermédiaire dudit tube, ce tube d'assistance respiratoire comportant un conduit de communication traversant la paroi dudit tube et établissant une communication entre l'intérieur dudit tube et l'intérieur dudit ballonnet.

Ainsi, le générateur de gaz pour le gonflage du ballonnet étant supprimé, le ballonnet, en place dans la trachée, se gonfle et se dégonfle au rythme de l'insufflation du gaz respiratoire. La pression d'appui exercée par le ballonnet contre les parois de la trachée n'est donc plus continue, mais cyclique. De plus, la pression de gonflage du ballonnet est égale à celle du gaz respiratoire qui, par définition, est choisie non traumatisante pour les poumons du patient. Grâce à ces deux constatations, on voit donc qu'un tel ballonnet gonflable ne peut pas être traumatisant pour la paroi de la trachée.

On remarquera de plus que, bien qu'avec un tel tube d'assistance respiratoire, l'étanchéité dans la trachée n'est pas continue, mais cyclique, il n'en résulte aucun inconvénient, puisque l'étanchéité est assurée pendant l'introduction du gaz respiratoire dans les poumons, ce qui évite les retours du gaz respiratoire vers l'extérieur.

L'objet de la présente invention est de perfectionner un tel tube d'assistance respiratoire connu pour en améliorer, à la fois, les performances et la sécurité.

A cette fin, selon l'invention, le tube d'assistance respiratoire, dont l'extrémité distale est destinée à être introduite dans la trachée d'un patient et est munie d'un ballonnet gonflable pour assurer l'étanchéité entre ledit tube d'assistance respiratoire et ladite trachée et dont l'extrémité proximale est destinée à être reliée à un respirateur artificiel pour amener du gaz respiratoire dans les poumons du patient, par l'intermédiaire dudit tube d'assistance respiratoire qui comporte au moins un premier conduit traversant la paroi dudit tube et établissant une communication entre le conduit intérieur dudit tube et la cavité intérieure dudit ballonnet, est caractérisé en ce qu'il comporte, en combinaison :
- des moyens de déflexion internes pour au moins un jet de gaz respiratoire véhiculé par au moins un premier conduit longitudinal pratiqué dans l'épaisseur dudit tube ;
- un second conduit traversant, reliant la cavité intérieure dudit ballonnet avec la partie dudit conduit intérieur dudit tube se trouvant entre lesdits moyens de déflexion internes et la face d'extrémité distale dudit tube ; et
- au moins un deuxiéme conduit longitudinal débouchant dans ladite cavité dudit ballonnet et destiné à relier ladite cavité à l'extérieur dudit patient.

Ainsi, lesdits moyens de déflexion internes améliorent la ventilation des patients, tandis que lesdits conduits traversants, associés audit deuxiéme conduit longitudinal, assurent une sécurité maximale. Ce deuxiéme conduit longitudinal peut être relié à un capteur de pression susceptible de contrôler le fonctionnement desdits moyens amenant le gaz respiratoire dans les poumons du patient. De préférence, ledit deuxième conduit longitudinal débouche, dans la cavité du ballonnet, au voisinage de l'extrémité la moins distale de celui-ci.

De préférence, afin d'évacuer d'éventuelles mucosités du ballonnet, lesdits conduits traversants se trouvent au voisinage de l'extrémité la plus distale dudit ballonnet. De plus, ii est avantageux, à ce propos, que lesdits conduits traversants soient inclinés par rapport audit tube, l'orifice par lequel lesdits conduits traversants débouchent dans le conduit intérieur dudit tube étant plus proche de la face d'extrémité distale de ce-lui-ci que l'orifice par lequel lesdits conduits traversants débouchent dans ledit ballonnet.

Pour améliorer encore la sécurité, on peut prévoir un autre conduit capillaire longitudinal débouchant en aval dudit ballonnet et relié à un autre capteur de pression apte à arrêter le fonctionnement dudit respirateur.

Ledit ballonnet gonflable peut être partagé en deux cavités, dans l'une desquelles débouchent ledit premier conduit traversant et ledit deuxième conduit longitudinal, et dans l'autre desquelles débouche un troisième conduit longitudinal, ménagé dans l'épaisseur dudit tube et apte à être relié à une source de gaz. On peut ainsi communiquer audit ballonnet une forme résiduelle non complètement aplatie lors du dégonflement de celui-ci.

De préférence, ledit second conduit traversant débouche dans la même cavité que ledit premier conduit traversant et ledit deuxième conduit longitudinal.

On remarquera que les documents US-A-5 235 973 et EP-A-074 809 décrivent des tubes d'assistance respiratoire pourvus d'un conduit longitudinal débouchant dans la cavité du ballonnet et destiné à relier la cavité de celui-ci à l'extérieur du patient. De plus, le document GB-A-1 171 439 décrit un tube respiratoire avec un ballonnet à double paroi.

Les figures du dessin annexé feront bien comprendre comment l'invention peut être réalisée. Sur ces figures, des références identiques désignent des éléments semblables.

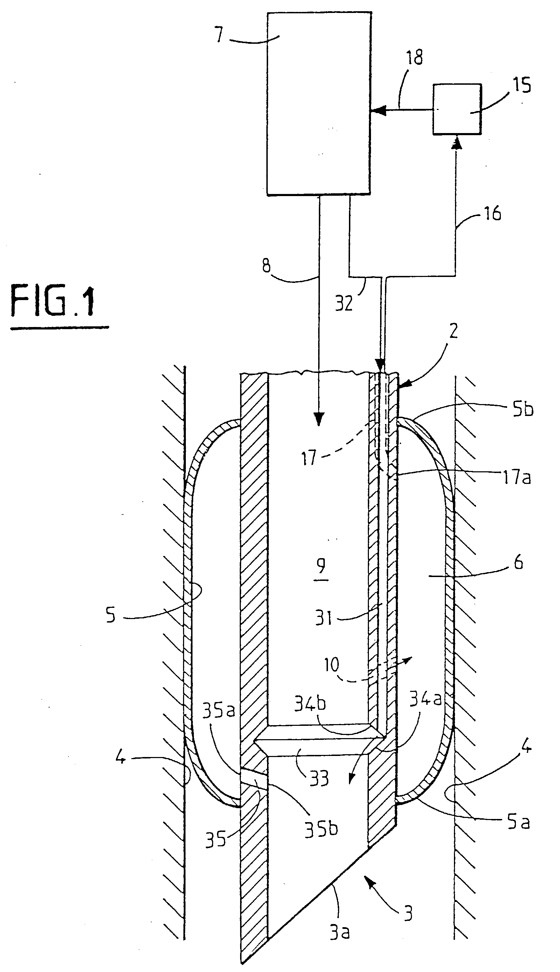

La figure 1 est un schéma synoptique d'une variante de réalisation du tube d'assistance respiratoire selon l'invention, mis en place dans une trachée. Sur cette figure, seule la partie distale desdit tube a été représentée à échelle agrandie, en association avec un respirateur artificiel.

L'appareil d'assistance respiratoire 1, montré par la figure 1, comprend un tube d'assistance respiratoire 2, conforme à la présente invention, dont l'extrémité distale 3, destinée à être introduite dans la trachée 4 d'un patient, est munie d'un ballonnet gonflable 5, définissant une cavité close 6 autour dudit tube 2. Le ballonnet 5 est à l'état dégonflé lorsqu'il est introduit dans la trachée 4 et, une fois en place dans celle-ci, il a pour objet principal, à l'état gonflé, d'obturer avec étanchéité l'espace annulaire compris entre l'extrémité distale 3 du tube 2 et la trachée 4, afin d'éviter le retour non contrôlé, vers l'extérieur du patient, du gaz respiratoire introduit dans les poumons dudit patient à travers ledit tube 2.

Un respirateur artificiel 7 est prévu pour insuffler un gaz respiratoire sous une pression cyclique dans lesdits poumons (non représentés, mais disposés vers le bas de la figure 1). Un tel gaz respiratoire peut être de l'oxygène ou un mélange de gaz contenant de l'oxygène, à une pression de crête par exemple égale à 3 bars. Le respirateur 7 commande les périodes insufflatoires (et éventuellement expiratoires) de l'assistance respiratoire du patient. Il est relié, par l'intermédiaire d'une liaison 8, au conduit intérieur 9 dudit tube d'assistance respiratoire 2.

Ledit conduit intérieur 9 du tube d'assistance respiratoire 2 est en communication avec la cavité intérieure 6 du ballonnet 5, par un conduit 10, traversant la paroi de la partie d'extrémité distale 3 du tube 2. De préférence, le conduit de communication 10 se trouve du côté de l'extrémité la plus distale 5a du ballonnet 5 et est incliné de façon que son orifice 10b, par lequel il débouche dans le conduit intérieur 9 du tube 2, soit plus proche de la face extrême 3a de l'extrémité distale 3 dudit tube que son orifice 10a, par lequel il débouche dans la cavité 6 du ballonnet 5.

A des fins de sécurité, on peut prévoir des moyens susceptibles d'interrompre le fonctionnement du respirateur en cas de surpression. Par exemple dans le mode de réalisation de la figure 1, on a prévu un capteur de pression 15 relié, par une liaison 16, à un conduit capillaire longitudinal 17, logé dans la paroi du tube 2 et débouchant en 17a dans la cavité 6 du ballonnet 5,de préférence du côté de l'extrémité la moins distale 5b de celui-ci. Par une liaison 18, le capteur de pression 15 est apte à arrêter le fonctionnement du respirateur 7.

Ainsi, lorsqu'après introduction du tube d'assistance respiratoire 2 dans la trachée 4, le ballonnet 5 étant à l'état dégonflé, on met en marche le respirateur 7, celui-ci introduit, avec une pression cyclique correspondant au rythme insufflatoire désiré, du gaz respiratoire dans le conduit intérieur 9 du tube 2, en direction des poumons du patient. Grâce au conduit traversant 10, la pression à l'intérieur de la cavité 6 du ballonnet 5 est continuellement égale à celle régnant dans ledit conduit intérieur 9. Par suite, le ballonnet 5 se gonfle et se dégonfle au rythme de la variation de pression du gaz respiratoire introduit par le respirateur 7 dans le tube 2. Le ballonnet 5 assure donc l'étanchéité dans la trachée 4, dés que du gaz respiratoire traverse le tube 2, en direction des poumons. En revanche, en l'absence du gaz respiratoire dans le tube 2, le ballonnet 5 se dégonfle.

Ainsi, on évite la pression continue, traumatisante pour la trachée, exercée par les ballonnets des tubes d'assistance respiratoire connus sur ladite trachée.

On remarquera que la pression maximale admise dans le ballonnet 5 est la pression de crête du gaz respiratoire et que, puisque cette pression de crête est évidemment choisie pour ne pas traumatiser les poumons, elle ne peut a fortiori pas léser la trachée, lorsqu'elle applique le ballonnet 5 contre cette dernière.

Par ailleurs, dans le mode de réalisation de la figure 1, on a prévu au moins un conduit longitudinal 31 logé dans la paroi du tube 2 et susceptible d'être relié à son extrémité proximale au respirateur 7 par l'intermédiaire d'une liaison 32. A son extrémité distale, le conduit 31 débouche dans un évidement annulaire 33, formé de surfaces coniques 34a et 34b, opposées et convergeant vers l'axe du tube 2. Lorsque le respirateur 7 adresse au conduit longitudinal auxiliaire 31 du gaz respiratoire sous pression à travers la liaison 32, le jet gazeux sortant de l'extrémité distale dudit conduit 31 heurte la face inclinée 34a, qui le défléchit en direction de l'axe dudit tube 2. Il en résulte une amélioration de la circulation gazeuse dans le conduit intérieur 9 du tube 2, ce qui favorise l'introduction, dans les poumons du patient, du gaz respiratoire amené dans le tube 2 par la liaison 8.

L'évidement annulaire 33 est plus proche de la face distale d'extrémité 3a que le conduit traversant 10.

De plus, dans la paroi du tube 2, est pratiqué un autre conduit traversant 35, débouchant en 35a à l'intérieur 6 du ballonnet 5 et en 35b à l'intérieur du tube 2, en aval (par rapport au gaz respiratoire insufflé) de l'évidement annulaire 33, et donc au voisinage de la face distale d'extrémité 3a.

Ainsi, si une surpression se produit dans le circuit pulmonaire du patient, elle est transmise à la cavité 6 du ballonnet 5 par le conduit traversant 35, puis détectée par le capteur 15, à travers le conduit 17 et la liaison 16. Le capteur 15 peut alors arrêter le fonctionnement du respirateur 7.

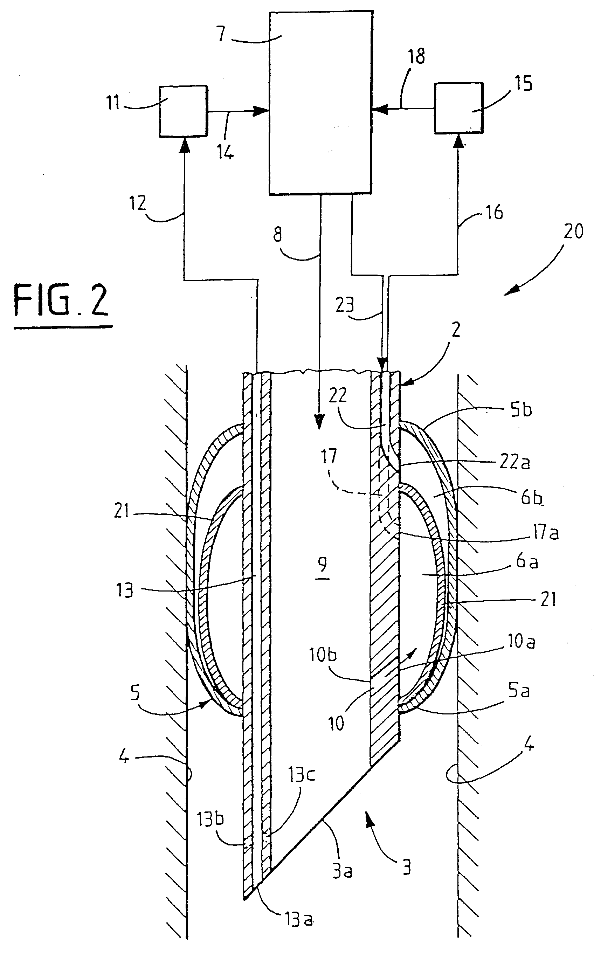

Dans la variante de réalisation 20 du tube d'assistance respiratoire représentée sur la figure 2, on retrouve tous les éléments 2 à 18 mentionnés ci-dessus à propos de l'appareil 1 de la figure 1.

Pour des raisons de redondance de sécurité, la variante de réalisation 20 de la figure 2 comporte, de plus, un autre capteur de pression 11 relié, par une liaison 12, à un conduit capillaire longitudinal 13, logé dans la paroi du tube 2 et débouchant en aval du ballonnet 5, par exemple en 13a dans la face extrême 3a, et/ou en 13b et en 13c dans les parois externe et interne dudit tube 2. Par une liaison 14, le capteur de pression 11 est apte à arrêter le fonctionnement du respirateur 7.

Cependant, dans cette variante de réalisation 20, le ballonnet 5 est partagé intérieurement en deux cavités closes 6a et 6b par une cloison souple 21.

La cavité 6a est pratiquement semblable à la cavité 6 de la figure 1, et les conduits 10 et 17 y débouchent d'une manière semblable à ce qui a été décrit ci-dessus.

Dans la cavité 6b, débouche, en 22a, un conduit capillaire longitudinal 22 relié par une liaison 23 au respirateur 7 et logé dans la paroi dudit tube.

Grâce à cette liaison 23, il est donc possible d'introduire dans la cavité 6b un gaz sous très faible pression, par exemple de l'ordre de 0,01 bar, de façon à communiquer à la cavité 6b une faible rigidité permanente pendant le fonctionnement du respirateur 7. Ainsi, lors des variations en baisse de la pression du gaz respiratoire amené par la liaison 8, seule la cavité 6a se dégonfle et le ballon 5 garde un volume résiduel non nul, dû à la cavité 6b sous faible pression. En revanche, lors des variations à la hausse de la pression du gaz respiratoire amené par la liaison 8, seule la cavité 6a (plus petite que la cavité 6) a à être gonflée pour assurer l'étanchéité au niveau de la trachée 4.

On voit donc que, grâce à la cloison 21, au conduit 22 et à la liaison 23, on assure le synchronisme entre les variations de pression du gaz respiratoire circulant dans le tube 2 et les mouvements de dilatation et de rétraction du ballonnet 5.

Bien entendu, il est possible de prévoir, pour le tube d'assistance respiratoire conforme à la présente invention, une pluralité de conduits, chacun correspondant à un ou plusieurs conduits 10, 17, 22 et/ou 31. Dans tous les cas, ces divers conduits sont répartis autour de l'axe du tube 2, de façon à ne pas interférer les uns avec les autres.

De plus, dans des applications particulières (par exemple, pour anesthésie au protoxyde d'azote) , on peut relier les conduits 22 et 17 ou 13 et 22 entre eux par l'intermédiaire d'une valve uni- ou bi-directionnelle.

Dans le cas d'utilisation de la sonde avec ventilateur classique, il faut prévoir un moyen de représsurisation de l'air prélevé sur le circuit du ventilateur.

## Revendications

1. Tube d'assistance respiratoire (2), dont l'extrémité distale (3) est destinée à être introduite dans la trachée (4) d'un patient et est munie d'un ballonnet gonflable (5) pour assurer l'étanchéité entre ledit tube d'assistance respiratoire (2) et ladite trachée (4) et dont l'extrémité proximale est destinée à être reliée à un respirateur artificiel (7) pour amener du gaz respiratoire dans les poumons du patient, par l'intermédiaire dudit tube d'assistance respiratoire (2) qui comporte au moins un premier conduit (10) traversant la paroi dudit tube (2) et établissant une communication entre le conduit intérieur (9) dudit tube (2) et la cavité intérieure (6) dudit ballonnet (5), un second conduit traversant (35), reliant la cavité intérieure (6) dudit ballonnet (5) avec la partie dudit conduit intérieur (9) dudit tube
caractérisé en ce qu'il comporte, en combinaison :
- des moyens de déflexion internes (33, 34a) pour au moins un jet de gaz respiratoire véhiculé par au moins un premier conduit longitudinal (31) pratiqué dans l'épaisseur dudit tube (2) ;
- ledit second conduit traversant (35) se trouvant entre lesdits moyens de déflexion internes (33, 34a) et la face d'extrémité distale (3a) dudit tube ; et
- au moins un deuxième conduit longitudinal (17) débouchant dans ladite cavité (6) dudit ballonnet et destiné à relier ladite cavité (6) à l'extérieur dudit patient.

2. Tube d'assistance respiratoire selon la revendication 1,
caractérisé en ce que ledit deuxième conduit longitudinal (17) est relié à un capteur de pression (15) susceptible de contrôler le fonctionnement dudit respirateur (7).

3. Tube d'assistance respiratoire selon l'une des revendications 1 ou 2,
caractérisé en ce que lesdits premier et second conduits traversants (10 et 35) se trouvent au voisinage de l'extrémité la plus distale (5a) du ballonnet (5), tandis que le deuxième conduit longitudinal (17) débouche au voisinage de l'extrémité la moins distale (5b) dudit ballonnet (5).

4. Tube d'assistance respiratoire selon l'une quelconque des revendications 1 à 3,
caractérisé en ce qu'il comporte un conduit capillaire longitudinal (13) débouchant en aval dudit ballonnet (15) et relié à un capteur de pression (11) apte à arrêter le fonctionnement dudit respirateur (7).

5. Tube d'assistance respiratoire selon l'une quelconque des revendications 1 à 4,
caractérisé en ce que ledit ballonnet gonflable (5) est partagé en deux cavités (6a et 6b), dans l'une desquelles (6a) débouchent ledit premier conduit traversant (10) et ledit deuxième conduit longitudinal (17), et dans l'autre desquelles (6b) débouche un troisième conduit longitudinal (22), ménagé dans l'épaisseur dudit tube et apte à être relié à une source de gaz.

6. Tube d'assistance respiratoire selon la revendication 5,
caractérisé en ce que ledit second conduit traversant (35), reliant la cavité (6) dudit ballonnet (5) avec la partie dudit conduit intérieur (9) dudit tube se trouvant entre lesdits moyens de déflexion internes (33, 34a) et la face d'extrémité distale (3a) dudit tube (2), débouche dans la mêne cavité (6a) que ledit premier conduit traversant (10) et ledit deuxième conduit longitudinal (17).

## Claims

1. Tube for respiratory assistance (2), whose distal end (3) is intended to be introduced into a patient's trachea (4) and is equipped with an inflatable balloon (5) to ensure leaktightness between the said tube for respiratory assistance (2) and the said trachea (4), and whose proximal end is intended to be connected to an artificial respirator (7) for conveying respiratory gas into the patient's lungs, by way of the said tube for respiratory assistance (2), which includes at least one first conduit (10) passing through the wall of the said tube (2) and establishing a communication between the internal conduit (9) of the said tube (2) and the internal cavity (6) of the said balloon (5), a second through-conduit (35) connecting the internal cavity (6) of the said balloon (5) to that part of the said internal conduit (9) of the said tube, characterized in that it includes, in combination:
- internal means of deflection (33, 34a) for at least one jet of respiratory gas conveyed via at least one first longitudinal conduit (31) formed within the thickness of the said tube (2);
- the said second through-conduit (35) being situated between the said internal means of deflection (33, 34a) and the distal end face (3a) of the said tube; and
- at least one second longitudinal conduit (17) opening into the said cavity (6) of the said balloon and intended to connect the said cavity (6) to the area outside the said patient.

2. Tube for respiratory assistance according to Claim 1, characterized in that the said second longitudinal conduit (17) is connected to a pressure sensor (15) which is able to control the functioning of the said respirator (7).

3. Tube for respiratory assistance according to one of Claims 1 or 2, characterized in that the said first and second through-conduits (10 and 35) are located in the vicinity of the most distal end (5a) of the balloon (5), while the second longitudinal conduit (17) opens out in the vicinity of the least distal end (5b) of the said balloon (5).

4. Tube for respiratory assistance according to any one of Claims 1 to 3, characterized in that it includes a longitudinal capillary conduit (13) opening out downstream of the said balloon (15) and connected to a pressure sensor (11) able to stop the functioning of the said respirator (7).

5. Tube for respiratory assistance according to any one of Claims 1 to 4, characterized in that the said inflatable balloon (5) is divided into two cavities (6a and 6b), the said first through-conduit (10) and the said second longitudinal conduit (17) opening into one (6a) of these cavities, and a third longitudinal conduit (22), formed within the thickness of the said tube and able to be connected to a gas source, opening into the other (6b) of these cavities.

6. Tube for respiratory assistance according to Claim 5, characterized in that the said second through-conduit (35), connecting the cavity (6) of the said balloon (5) to that part of the said internal conduit (9) of the said tube located between the said internal means of deflection (33, 34a) and the distal end face (3a) of the said tube (2), opens into the same cavity (6a) as the said first through-conduit (10) and the said second longitudinal conduit (17).

## Patentansprüche

1. Tubus zur Atmungsunterstützung (2), dessen distales Ende (3) dazu bestimmt ist, in die Trachea (4) eines Patienten eingeführt zu werden und mit einem aufblasbaren Ballon (5) versehen ist, um Abdichtung zwischen dem Tubus zur Atmungsunterstützung (2) und der Trachea (4) sicherzustellen und wobei das proximale Ende dazu bestimmt ist, mit einem künstlichen Respirator (7) verbunden zu sein, um respiratorisches Gas in die Lungen des Patienten mittels des Tubus zur Atmungsunterstützung (2) zu bringen, der mindestens eine erste Leitung (10), die die Wand des Tubus (2) durchquert und eine Verbindung zwischen der Innenleitung (9) des Tubus (2) und dem Innenhohlraum (6) des Ballons (5) herstellt, aufweist; wobei eine zweite durchquerende Leitung (35) den Innenhohlraum (6) des Ballons (5) mit dem Teil der Innenleitung (9) des Tubus (2) verbindet, dadurch gekennzeichnet, daß er in Kombination aufweist:
- Mittel zur internen Ablenkung (33, 34 a) für mindestens einen Strom respiratorischen Gases, der durch mindestens eine erste longitudinalen Leitung (31), die in der Dicke des Tubus (2) verläuft, gefördert wird;
- die zweite durchquerende Leitung (35), die sich zwischen den Mitteln zur internen Ablenkung (33, 34a) und der distalen Endfläche (3a) des Tubus (2) befindet; und
- mindestens eine zweite longitudinale Leitung (17), die im Hohlraum (6) des Ballons mündet und dazu bestimmt ist, den Hohlraum (6) mit dem Äußeren des Patienten zu verbinden.

2. Tubus zur Atmungsunterstützung nach Anspruch 1, dadurch gekennzeichnet, daß die zweite longitudinale Leitung (17) mit einem Druckaufnehmer (15) verbunden ist, der dazu befähigt ist, die Funktion des Respirators (7) zu steuern.

3. Tubus zur Atmungsunterstützung nach einem der Ansprüche 1 oder 2, dadurch gekennzeichnet, daß die ersten und zweiten durchquerenden Leitungen (10 und 35) sich in der Nähe des entferntesten distalen Endes (5a) des Ballons (5) befinden, wobei die zweite longitudinale Leitung (17) in der Nähe des nächsten distalen Endes (5b) des Ballons (5) mündet.

4. Tubus zur Atmungsunterstützung nach irgendeinem der Ansprüche 1 bis 3, dadurch gekennzeichnet, daß er eine kapillare longitudinale Leitung (13) umfaßt, die stromabwärts des Ballons (5) mündet und mit einem Druckaufnehmer (11) verbunden ist, der die Funktion des Respirators (7) abstellen kann.

5. Tubus zur Atmungsunterstützung nach irgendeinem der Ansprüche 1 bis 4, dadurch gekennzeichnet, daß der aufblasbare Ballon (5) in zwei Hohlräume (6a und 6b) geteilt ist, wobei in einem derselben (6a) die erste durchquerende Leitung (10) und die zweite longitudinale Leitung (17) münden und in dem anderen derselben (6b) eine dritte longitudinale Leitung (22) mündet, die in der Dicke des Tubus verläuft und mit einer Gasquelle verbunden werden kann.

6. Tubus zur Atmungsunterstützung nach Anspruch 5, dadurch gekennzeichnet, daß die zweite durchquerende Leitung (35), die den Hohlraum (6) des Ballons (5) mit dem Teil der Innenleitung (9) des Tubus verbindet, der sich zwischen den Mitteln zur internen Ablenkung (33, 34a) und der distalen Endfläche (3a) des Tubus (2) befindet, in denselben Hohlraum (6a) wie die erste durchquerende Leitung (10) und die zweite longitudinale Leitung (17) mündet.
